# EUROPÄISCHE PATENTSCHRIFT

(11) **EP 3 130 309 B1**
(45) Veröffentlichungstag und Bekanntmachung des Hinweises auf die Patenterteilung: **22.08.2018**
(21) Anmeldenummer: 15177339.7
(22) Anmeldetag: 17.07.2015
(51) Int. Cl.: A61C 5/62, A61C 5/00, B05C 17/005

(54) **DENTALAPPLIKATOR**
DENTAL APPLICATOR
APPLICATEUR DENTAIRE

(43) Veröffentlichungstag der Anmeldung: 15.02.2017
(73) Patentinhaber: Ivoclar Vivadent AG, 9494 Schaan (LI)
(72) Erfinder: MÜLLER, Frank, 6800 Feldkirch (AT)
(74) Vertreter: Baronetzky, Klaus

(56) Entgegenhaltungen:
- EP-A1- 2 630 931
- CN-A- 1 509 773
- FR-A1- 2 785 813
- US-A- 4 863 433
- US-A- 5 183 415

## Beschreibung

Die Erfindung betrifft einen Dentalapplikator, gemäß dem Oberbegriff von Anspruch 1.

Für die Applikation von fließfähigen Dentalmaterialien ist es bekannt, Dentalapplikatoren zu verwenden. Dentalmaterialien sind häufig recht hochwertig, und die genaue Dosierung des zu applizierenden Dentalmaterials ist daher nicht nur aus zahnmedizinischen, sondern aus wirtschaftlichen Gründen geboten.

Häufig werden daher Dentalmaterialien in einem Applikatorgehäuse mit Stempel bereit gehalten, wobei die genaue Menge des zu applizierenden Dentalmaterials mit Hilfsmitteln, wie beispielsweise einer Skala vorgegeben wird.

Das Dentalmaterial muss häufig an schlecht zugänglichen Stellen, wie beispielsweise einem Zahnwurzelkanal, appliziert werden. Hierzu wird dann typischerweise eine Kanüle verwendet, die einen Anschlusskörper und ein Kanülenröhrchen aufweist. Das Kanülenröhrchen hat einen ausgesprochen geringen Durchmesser von beispielsweise 0,8 mm, wobei der Durchmesser aber auch demgegenüber stark unterschiedlich sein kann.

Die Kanüle ist dann von dem Applikatorgehäuse trennbar.

Um den Erfordernissen der Applikation Rechnung zu tragen, bieten die Hersteller derartiger Dentalapplikatoren Kanülen mit Kanülenröhrchen mit unterschiedlicher Gestaltung an. Beispiele hierfür sind gerade Kanülenröhrchen, rund gebogene, gekröpfte, aber auch abgewinkelte Kanülenröhrchen.

In manchen Fällen muss nun der Zahnarzt mit dem gleichen Dentalmaterial im Mund des Patienten das Dentalmaterial an unterschiedlichen Orten applizieren. Bislang musste er hierfür die Kanüle mit dem zugehörigen Kanülenröhrchen wechseln, beispielsweise von gerade auf abgewinkelt. Hiermit ging nicht nur eine gewisse Menge an Dentalmaterial
verloren, sondern es ergibt sich bislang die Notwendigkeit, zwei unterschiedliche Kanülen bereitzuhalten.

Die FR 2 785 813 A1 offenbart eine eine trichterförmige Bohrung aufweisende Endabdeckung einer Injektionsvorrichtung, in die eine einen Sockel aufweisende Zweipunktnadel in die Bohrung einführbar und mittels eines Gewindes fixierbar ist.

Die US 4 863 433 B1 offenbart eine einen Behälter und einen Stempel mit einem inne

Die CN 1509773 A offenbart eine Vorrichtung mit einem einen Stempel beinhaltenden Behälter, wobei an diesem ein weiterer Behälter zur Lagerung einer Nadel angeordnet ist.

Demgegenüber liegt der Erfindung die Aufgabe zu Grunde, einen Dentalapplikator gemäß dem Oberbegriff von Anspruch 1 zu schaffen, der universeller verwendbar ist, also eine Anpassung an unterschiedliche Applikationssituationen ermöglicht, aber dennoch in gesamtwirtschaftlicher Betrachtung Einsparungen ermöglicht.

Diese Aufgabe wird erfindungsgemäß durch Anspruch 1 gelöst. Vorteilhafte Weiterbildungen ergeben sich aus den Unteransprüchen.

Erfindungsgemäß besonders günstig ist es, dass das Applikatorgehäuse und/oder der Stempelkörper selbst, also ein Teil des Dentalapplikators, es ermöglicht, die Form des Kanülenröhrchens erstmals an die unterschiedlichen Erfordernisse des Applikationsorts anzupassen.

Hierzu ist das Kanülenröhrchen biegsam ausgebildet, beispielsweise aus Stahl mit einer Wandstärke von 80 µm bei einem Durchmesser von 0,8 mm und einer Gesamtlänge von 25 mm.

Das erfindungsgemäße Biegen erfolgt bevorzugt, aber nicht unbedingt, mit einer Biegehilfe, die den Biegeradius vorgibt. Ein derartiges Stahlröhrchen lässt sich ohne Weiteres mit einem Biegeradius von weniger als 10 mm, beispielsweise von 5 mm, biegen.

Wenn nun das Ende, also beispielsweise die vordersten 3 mm des Kanülenröhrchens in die Aufnahmevorrichtung eingesetzt wird und eine Biegekraft ausgeübt wird, erfolgt typischerweise bis zunächst eine elastische, und dann eine plastische Verformung mit einem Biegeradius, der sich über den Verlauf des Röhrchens im Wesentlichen gleichförmig einstellt. Erst wenn der zulässige Biegeradius unterschritten ist, und an einer Stelle das Röhrchen gleichsam abknickt, erfolgt eine Einschnürung, und eine Anwendung des Röhrchens ist nicht mehr möglich. Dies wird jedoch erfindungsgemäß durch das vergleichsweise lange Kanülenröhrchen mit einer Länge von mindestens 10 mm und den vergleichsweise geringen Röhrenaußendurchmesser mit einer Dicke von höchstens 1 , 5 mm verhindert, aber auch dadurch, dass beispielsweise über einen Anschlag oder dergleichen die maximale Verbiegung begrenzt wird.

Erfindungsgemäß ist es in vorteilhafter Ausgestaltung dem Zahnarzt möglich, die erzielte Biegeform dem gewünschten Ergebnis anzupassen. Wenn der Biegeradius beispielsweise eher anschlusskörpernah ausgebildet werden soll, schiebt der Zahnarzt beim Biegen das Applikatorgehäuse in Richtung der Aufnahmeöffnung bzw. leicht jenseits der Aufnahmevorrichtung. Wenn andererseits der Biegeradius nahe dem Ende des Kanülenröhrchens erzeugt werden soll, zieht der Zahnarzt beim Biegen das Applikatorgehäuse leicht zurück, so dass je die erwünschte Form entsteht.

Auf diese Weise ist es der erfindungsgemäßen Biegehilfe auch möglich, einen im Wesentlichen S-förmigen Verlauf des Kanülenröhrchens bereitzustellen, wenn dies erwünscht ist, Hierzu wird beim Biegen zunächst einmal gezogen, dann wird das Applikatorgehäuse um 180 ° gedreht, und dann wird beim Biegen gedrückt.

Besonders günstig ist es, dass die Aufnahmevorrichtung in erfindungsgemäß vorteilhafter Ausgestaltung einen Tiefenanschlag aufweist. Hierdurch lässt sich die Hebelkraft, die beim Biegen ausgeübt wird, von vornherein einstellen. Wenn der Tiefenanschlag beispielsweise in 3 mm Tiefe liegt, treten die vorderen 3 mm des Kanülenröhrchens in die Aufnahmevorrichtung ein, und die Länge des Biegehebels beträgt dann ebenfalls
3 mm, was bei einem Röhrchendurchmesser von 1 mm ausreichend ist.

Es versteht sich, dass dickere Kanülenröhrchen bevorzugt auch tiefere Tiefenanschläge und dünnere weniger tiefe Tiefenanschläge aufweisen können. Auch kann ein Dentalapplikator mehrere Aufnahmevorrichtungen mit unterschiedlichen Durchmessern, in Abhängigkeit von der aufgesteckten Kanüle und deren Röhrchen haben.

Bevorzugt ist das Kanülenröhrchen mit seinem Ende mit einem geringen Spiel von beispielsweise 20 µm oder 50 µm in die Aufnahmevorrichtung eingesteckt. Das Kanülenröhrchen lässt sich nach dem Biegen dann leicht lösen, aber es erfolgt dennoch eine sichere Biegeführung.

Die Aufnahmevorrichtung kann bei einem aus Spritzguß hergestellten Applikatorgehäuse oder Stempelgehäuse unmittelbar in dieses integriert sein, beispielsweise durch eine entsprechende Sacklochbohrung.

Es ist aber auch möglich, eine entsprechende Buchse aus einem geeigneten Material separat herzustellen und in eine entsprechende Ausnehmung in dem Applikatorgehäuse oder in dem Stempelkörper einzustecken. Diese Ausgestaltung ist dann bevorzugt, wenn für unterschiedliche Durchmesser der Kanülen die gleichen Applikatorgehäuse und Stempelkörper bereitgehalten werden sollen, aber im Nachhinein - beispielsweise materialabhängig eine Festlegung erfolgen soll.

Hierzu wird dann bei der Verpackung die entsprechende Aufnahmevorrichtung-Buchse mit dem Applikatorgehäuse und dem Stempelkörper passend zu der Kanüle beigepackt.

Es ist insofern auch möglich, die Aufnahmevorrichtungs-Buchse mit einer hierzu passenden Kanüle zu verpacken und zu liefern.

Bevorzugt sind die erfindungsgemäßen Kanülenröhrchen im Lieferzustand gerade und lassen sich dann mit der erfindungsgemäßen Aufnahmevorrichtung in eine beliebige Form biegen.

In vorteilhafter Ausbildung ist die Aufnahmevorrichtung deutlich tiefer als es dem Erfordernis des eigentlichen Biegevorgangs entspricht. An einen geraden Abschnitt schließt sich ein gebogener Abschnitt von mehreren Millimetern Länge an. Das Biegen erfolgt nun so, dass sich das Biegeröhrchen an einer beliebigen Stelle an dem gebogenen Abschnitt anlehnt und daher der dortige Biegeradius vorgegeben ist. Dies hat den Vorteil, dass der minimale zulässige Biegeradius auf keinen Fall unterschritten ist.

In vorteilhafter Ausgestaltung ist es vorgesehen, die Aufnahmevorrichtung an einer beliebigen geeigneten Stelle des Applikatorgehäuses und/oder des Stempelkörpers vorzusehen, die eine entsprechende Wandstärke aufweist. Dies hat den Vorteil, dass keine zusätzliche Auswölbung oder dergleichen dort vorgesehen sein muss. Eine nennenswerte Schwächung der Struktur des Stempelkörpers und/oder des Applikatorgehäuses ist hiermit nicht verbunden.

So lässt sich die Aufnahmevorrichtung beispielsweise ohne Weiteres an der Rückenplatte oder Griffplatte des Applikatorgehäuses im Wesentlichen radial realisieren. Eine weitere Möglichkeit der Anbringung ist die Druckplatte des Stempelkörpers, wobei hier die Möglichkeit der radialen Anordnung besteht. Es lassen sich auch mehrere Aufnahmevorrichtungen mit geeigneten Durchmessern an der geeigneten Stelle anbringen, und es ist auch möglich, bei einer radialen Anordnung eine Winkelskala zu realisieren, die Biegung des Kanülenröhrchens andeuten kann.

Weitere Vorteile, Einzelheiten und Merkmale ergeben sich aus der nachfolgenden Beschreibung mehrerer Ausführungsbeispiele der Erfindung anhand der Zeichnung.

Es zeigen:
Fig. 1 eine schematische Seitenansicht einer Ausführungsform eines erfindungsge-mäßen Dentalapplikators;
- Fig. 2 die Ausführungsform gemäß Fig. 1, jedoch unter Darstellung des Applikatorge- . häuses mit abgenommener Kanüle, in der Ansicht von unten;
Fig. 3 eine perspektivische schematische Ansicht einer weiteren Ausführungsform eines Dentalapplikators; und
Fig. 4 ein Detail einer weiteren Ausführungsform eines erfindungsgemäßen Dentalapplikators.

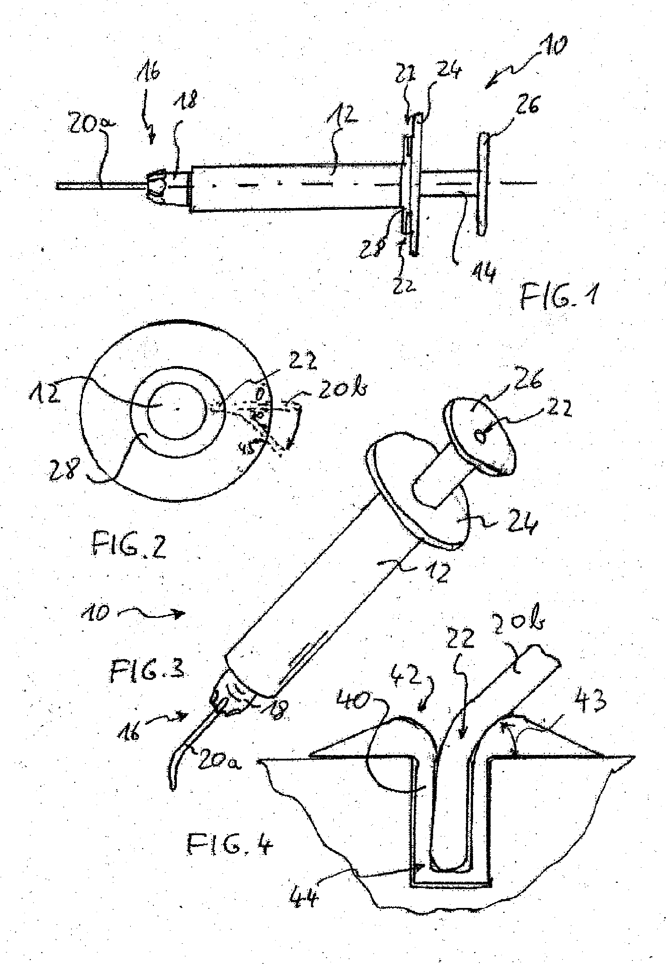

In Fig. 1 ist ein Dentalapplikator 10 schematisch in einer Ausführungsform dargestellt. Der Dentalapplikator weist ein Applikatorgehäuse 12 auf, in dem in an sich bekannter Weise ein Stempelkörper 14 geführt ist. Die Führung kann beispielsweise eine Schiebeführung nach der Art einer Spritze sein. Sie kann aber auch ein Gewinde aufweisen, so dass durch Drehen des Stempelkörpers unter hohem Druck Material aus dem Applikatorgehäuse herausgedrückt werden kann.

Der Dentalapplikator 10 weist ferner eine Kanüle 16 auf, die an dem vorderen Ende des Applikatorgehäuses lösbar befestigt ist. Die Befestigung erfolgt beispielsweise über ein Schraubgewinde oder beispielsweise einen Bajonettverschluss. In an sich bekannter Weise ist die Befestigung so ausgebildet, dass eine Abdichtung erfolgt, so dass unter Druck stehendes Dentalmaterial, das sich in dem Applikatorgehäuse 14 befindet, in die Kanüle 16 hinein gedrückt wird.

Die Kanüle 16 besteht aus einem Anschlusskörper 18 und einem Kanülenröhrchen 20a. Der Anschlusskörper 18 ist bevorzugt wie das Applikatorgehäuse 12 und der Stempelkörper 14 aus einem hierfür geeigneten Kunststoff. Hingegen ist erfindungsgemäß das Kanülenröhrchen 20b biegsam. Das Kanülenröhrchen 20b ist insofern bei entsprechenden Biegekräften plastisch verformbar, kann aber zusätzlich im geringeren Umfang auch elastisch verformbar sein.

Es ist bevorzugt recht dünnwandig mit einer Wandstärke zwischen beispielsweise 50 µm und 200 µm und einem Durchmesser von beispielsweise 1 mm. Die Länge kann zwischen 1 cm und beispielsweise 3 cm betragen. Es kann aus Stahl oder einem beliebigen anderen geeigneten Metall bestehen, das jedenfalls gegen das zu applizierende Dentalmaterial beständig ist.

Erfindungsgemäß weist der Dentalappliator im Bereich Applikatorgehäuse-Stempelkörper eine Aüfnahmevorrichtung 22 auf. Im dargestellten Ausführungsbeispiel sind zwei Aufnahmevorrichtungen 22 leicht schematisch dargestellt. Sie erstrecken sich nach der Art von Sacklöchern parallel zu einer Griffplatte 24 des Applikatorgehäuses. Die Griffplatte 24 dient dazu, das Applikatorgehäuse nach rückwärts abzuschließen und ist zugleich eine Greifhandhabe. Sie wird typischerweise mit zwei Fingern, beispielsweise mit dem Zeigefinger und dem Mittelfinger, hintergriffen, während mit dem Daumen Druck auf eine Druckplatte 26 ausgeübt wird, die den Stempelkörper 14 nach hinten abschließt.

Die Aufnahmevorrichtungen 22 erstrecken sich als Sacklöcher in radialer Richtung. Sie sind an einer Ringverstärkung 28 angebracht, die sich an die Griffplatte 24 in Richtung Kanüle 16 anlehnt und ebenfalls als Greifhandhabe dient. Der Durchmesser einer Aufnahmevorrichtung beträgt 1,25 mm und ist insofern für die Aufnahme von Kanülenröhrchen 20b mit einem Durchmesser von 1,2 mm bestimmt. Demgegenüber beträgt der Durchmesser der anderen Aufnahmevorrichtung 1,05 mm und ist für die Aufnahme von Kanülenröhrchen mit einem Durchmesser von 1,0 mm bestimmt. Die Tiefe der betreffenden Aufnahmevorrichtung, die nach der Art einer Sacklochbohrung ausgebildet ist, beträgt 3,5 mm bzw. 3 mm.

Dieses Ausführungsbeispiel erlaubt eine schlanke Ausgestaltung des erfindungsgemäßen Dentalapplikators. Zur Vornahme des Biegevorgangs wird das Kanülenröhrchen 20b abgenommen und in die passende Aufnahmevorrichtung 22 eingesteckt und lässt sich dann nach Belieben verbiegen.

Nach dem Biegevorgang wird die Kanüle 16 mit dem nunmehr gebogenen Kanülenröhrchen 20b wieder mit dem Anschlusskörper 18 an dem Applikatorgehäuse 12 angebracht.

Aus Fig. 2 ist ersichtlich, dass die Griffplatte 24 Markierungen tragen kann, die den Biegevorgang numerisch unterstützen. Hier sind die Markierungen 0°, 30° und 45°, entsprechend einer Abbiegung um den je genannten Winkel, eingetragen. Der Benutzer kann so beim Einstecken in die Aufnahmevorrichtung 22 des Applikatorgehäuses 12 bzw. des Ringverstärkung 28 ersehen, um welchen Winkel er die Biegung gerade vornimmt.

In einer weiteren, hier nicht dargestellten Ausführungsform, sind Anschläge vorgesehen, die es erlauben, den Biegevorgang zu begrenzen und dem Benutzer auch zugleich einen Hinweis zu geben, ab welchem Biegewinkel eine Einschnürung des Kanülenröhrchens 20b zu befürchten wäre.

Die Erfindung ist nicht auf die Anordnung der Aufnahmevorrichtung 22 an dem Applikatorgehäuse 12 beschränkt. Die Aufnahmevorrichtung an einer beliebigen geeigneten Stelle, auch irgendwo an der Wand oder der Stirnseite des Applikatorgehäuses 12 angebracht sein. Fig. 3 zeigt keinen erfindungsgemäßen Dentalapplikator. Gemäß Fig. 3 ist sie zentral in dem Stempelkörper 14 in der Druckplatte 26 angebracht. Auch hier gilt, dass bei Entfernen des Anschlusskörpers 18 von dem Applikatorgehäuse 12 und Einführen des Kanülenröhrchens 20b mit seiner vorderen Spitze in die Aufnahmevorrichtung 22 ein Verbiegen möglich ist. Gemäß Fig. 3 hat das Kanülenröhrchen 20a bereits eine gebogene Spitze.

Aus Fig. 4 ist die Ausgestaltung einer Aufnahmevorrichtung 22 im Detail ersichtlich. Hier ist die Aufnahmevorrichtung 22 als Buchse 40 ausgebildet, wobei es sich versteht, dass eine entsprechende Form auch bei integrierter, also einstückiger Realisierung möglich ist.

Das nach außen weisende Ende 42 der Aufnahmevorrichtung 22 weist einen Radius auf, der zugleich einen Biegeanschlag bildet. Das Kanülenröhrchen 20b lässt sich so entlang des gebogenen Endes 42 mit einem vorgegebenen Biegeradius verbiegen. Die gebogene Endfläche 42 ist kreisringförmig, so dass ein Verbiegen in eine beliebige Richtung möglich ist. Eine Ausgestaltung des Biegeendes mit vordefinierten Rampenwinkeln 43 ist ebenfalls denkbar.

Wie aus Fig. 4 ersichtlich ist, bildet die Aufnahmevorrichtung 22 zugleich einen Tiefenanschlag 44. Das Kanülenröhrchen 20b lässt sich bis zum Boden der Aufnahmevorrichtung einführen, also, bis die Spitze des Kanülenröhrchens 20b in der Aufnahmevorrichtung am Boden der Aufnahmevorrichtung anliegt.

In dieser Position lässt sich das Kanülenröhrchen 20b bzw. genauer gesagt dessen vorderes Ende in beliebiger Weise geeignet verbiegen.

In dem dargestellten Ausführungsbeispiel gemäß Fig. 4 ist die Buchse 40 austauschbar, so dass auch Buchsen mit Aufnahmevorrichtungen 22 mit einem beliebigen anderen geeigneten Innendurchmesser anstelle dessen einsetzbar sind.

## Patentansprüche

1. Dentalapplikator, mit einem Applikatorgehäuse (12) und einem Stempelkörper (14), der in dem Applikatorgehäuse (12) beweglich geführt ist, wobei das Applikatorgehäuse (12) einen Anschluss für die lösbare und druckfeste Montage einer Kanüle (16) aufweist, welche Kanüle (16) einen Anschlusskörper (18) und ein Kanülenröhrchen (20) aufweist, das in oder an dem Anschlusskörper (18) fest verbunden ist, wobei das Applikatorgehäuse (12) und/oder der Stempelkörper (14), eine Aufnahmevorrichtung (22) für die mindestens teilweise Aufnahme eines biegsam ausgebildeten Kanülenröhrchens (20) aufweist, **dadurch gekennzeichnet, dass** die Aufnahmevorrichtung (22) als sich radial erstreckende Ausnehmung an dem Stempelkörper (14) oder an dem Applikatorgehäuse (12) ausgebildet ist.

2. Dentalapplikator nach Anspruch 1, **dadurch gekennzeichnet, dass** die Aufnahmevorrichtung (22) an einer Griffplatte (24) des Stempelkörpers (14) oder des Applikatorgehäuses (12) ausgebildet ist.

3. Dentalapplikator nach Anspruch 1 oder 2, **dadurch gekennzeichnet, dass** die Ausnehmung sich je der zugehörigen Griffplatte (24) benachbart, wegerstreckt, und dass an der der Ausnehmung benachbarten Griffplatte (24) Winkelmarkierungen angebracht sind, die den Biegewinkel des Kanülenröhrchens (20) beim Verbiegen des Endes des Kanülenröhrchens (20), das in die Aufnahmevorrichtung (22) eingesteckt ist, signalisieren.

4. Dentalapplikator nach Anspruch 1, **dadurch gekennzeichnet, dass** die Aufnahmevorrichtung (22) eine Ausnehmung an dem Applikatorgehäuse (12) und/oder dem Stempelkörper (14) ist, in welche das Kanülenröhrchen (20) teilweise einsteckbar ist.

5. Dentalapplikator nach einem der vorhergehenden Ansprüche, **dadurch gekennzeichnet, dass** die Aufnahmevorrichtung (22), insbesondere die Ausnehmung, einen Tiefenanschlag (44) aufweist und dass das Kanülenröhrchen (20) in die Aufnahmevorrichtung (22) bzw. die Ausnehmung bis zur Anlage an dem Tiefenanschlag (44) einsteckbar ist.

6. Dentalapplikator nach einem der vorhergehenden Ansprüche, **dadurch gekennzeichnet, dass** eine Ausnehmung der Aufnahmevorrichtung (22) eine Tiefe hat, die mindestens das Doppelte, bevorzugt mindestens das Dreifache und besonders bevorzugt höchstens das Zehnfache des Durchmessers des Kanülenröhrchens (20) beträgt.

7. Dentalapplikator nach einem der vorhergehenden Ansprüche, **dadurch gekennzeichnet, dass** der Dentalapplikator ein Kanülenröhrchen (20) umfasst und das Kanülenröhrchen (20) einen Durchmesser aufweist, der eine Spielführung mit einem Spiel von mindestens 50µm in der Ausnehmung ermöglicht, und die Ausnehmung insofern einen um mindestens 50µm größeren Innendurchmesser als den Außendurchmesser des Kanülenröhrchens (20) aufweist.

8. Dentalapplikator nach einem der vorhergehenden Ansprüche, **dadurch gekennzeichnet, dass** die Aufnahmevorrichtung (22) mindestens zwei Biegeanschläge aufweist, die voneinander beabstandet sind, insbesondere entsprechend der Eindringtiefe des Kanülenröhrchens (20) in der Aufnahmevorrichtung (22), sind, welche Biegeanschläge gegen-einander um 180° versetzt sind.

9. Dentalapplikator nach einem der vorhergehenden Ansprüche, **dadurch gekennzeichnet, dass** die Aufnahmevorrichtung (22) eine Ausnehmung aufweist, kreisförmig ist und gerade verläuft.

10. Dentalapplikator nach einem der vorhergehenden Ansprüche, **dadurch gekennzeichnet, dass** die Aufnahmevorrichtung (22) an ihrem nach außen weisenden Ende mindestens eine gebogene Endfläche (42) aufweist, die sich insbesondere kreisförmig gebogen um das nach außen weisende Ende (42) herum erstreckt.

11. Dentalapplikator nach Anspruch 10, **dadurch gekennzeichnet, dass** die gebogene Endfläche (42) einen Radius aufweist, der mindestens dem minimal zulässigen Biegeradius des Kanülenröhrchens (20) entspricht.

12. Dentalapplikator nach Anspruch 10, **dadurch gekennzeichnet, dass** die gebogene Endfläche (42) im Auslauf des Radius mindestens einen vordefinierte Rampenwinkel (43) aufweisen kann.

## Claims

1. A dental applicator having an applicator housing (12) and a stamp body (14), which is movably guided in the applicator housing (12), wherein the applicator housing (12) comprises a connection for releasably and pressure-resistantly mounting a cannula (16), the cannula (16) comprising a connection body (18) and a cannula tube (20), which is fixedly connected in or at the connection body (18), wherein the applicator housing (12) and/or the stamp body (14) comprises a receiving device (22) for at least partially receiving a flexibly formed cannula tube (20), **characterized in that** the receiving device (22) is formed as a radially extending recess at the stamp body (14) or at the applicator housing (12).

2. The dental applicator according to Claim 1, **characterized in that** the receiving device (22) is formed at a grip plate (24) of the stamp body (14) or the applicator housing (12).

3. The dental applicator according to Claim 1 or 2, **characterized in that** the recess extends away from the respective associated grip plate (24), and **in that** angle marks are attached at the grip plate (24) adjacent to the recess, the angle marks signalizing the bend angle of the cannula tube (20) when bending the end of the cannula tube (20), which is inserted into the receiving device (22).

4. The dental applicator according to Claim 1, **characterized in that** the receiving device (22) is a recess at the applicator housing (12) and/or the stamp body (14), into which the cannula tube (20) is partially insertable.

5. The dental applicator according to one of the preceding Claims **characterized in that** the receiving device (22), especially the recess, comprises a depth stop (44) and **in that** the cannula tube (20) is insertable into the receiving device (22) or the recess, respectively, until abutting the depth stop (44).

6. The dental applicator according to one of the preceding Claims, **characterized in that** a recess of the receiving device (22) has a depth, which is at least twofold, preferably threefold and especially preferable at most tenfold as much as the diameter of the cannula tube (20).

7. The dental applicator according to one of the preceding Claims, **characterized in that** the dental applicator comprises a cannula tube (20), and the cannula tube (20) comprises a diameter, which allows play control with a play of at least 50 µm in the recess, and as such the recess comprises an inner diameter at least 50 µm larger than the outer diameter of the cannula tube (20).

8. The dental applicator according to one of the preceding Claims, **characterized in that** the receiving device (22) comprises at least two bending stops, which are spaced apart from each other, and especially are spaced apart from each other in correspondence to the penetration depth of the cannula tube (20) in the receiving device (22), which bend stops are offset to each other by 180°.

9. The dental applicator according to one of the preceding Claims, **characterized in that** the receiving device (22) comprises a recess, is circular and runs straight.

10. The dental applicator according to one of the preceding Claims, **characterized in that** the receiving device (22), at its outwardly facing end, comprises at least a bent end face (42), which especially extends circularly bent around the outwardly bent end.

11. The dental applicator according to Claim 10, **characterized in that** the bent end face (42) comprises a radius, which at least corresponds to the minimally allowable bending radius of the cannula tube (20).

12. The dental applicator according to Claim 10, **characterized in that** the bent end face (42) may comprise at least one predefined ramp angle (43) in the outlet of the radius.

## Revendications

1. Applicateur dentaire avec un boitier d'applicateur(12) et un corps de poinçon (14), qui est guidé de manière mobile dans le boitier d'applicateur (12), où le boîtier d'applicateur(12) dispose d'une connexion pour le montage de manière amovible et résistante à la compression d'une canule (16), ladite canule (16) ayant un corps de connecteur (18) et un tube de canule (20) qui est fermement fixé dans ou sur le corps de connecteur (18), où le boitier d'applicateur (12) et/ou le corps de poinçon (14) présente un dispositif de réception (22) pour la réception au moins partielle d'un tube de canule (20) formé flexible, **caractérisé en ce que** le dispositif de réception (22) est formé comme évidement s'étendant radialement sur le corps de poinçon (14) ou le boitier de l'applicateur(12).

2. Applicateur dentaire selon la revendication 1, **caractérisé en ce que** le dispositif de réception (22) est formé sur une plaque de préhension (24) du corps de poinçon (14) ou du boitier de l'applicateur (12).

3. Applicateur dentaire selon la revendication 1 ou 2, **caractérisé en ce que** l'évidement part adjacent à la plaque de préhension (24) associée, et que la plaque de préhension (24) adjacente à l'évidement porte des marqueurs d'angle, qui signalent l'angle de flexion du tube de canule (20) lors de la flexion de l'extrémité du tube de canule (20), qui est inséré dans le dispositif de réception (22).

4. Applicateur dentaire selon la revendication 1, **caractérisé en ce que** le dispositif de réception (22) est un évidement sur le boitier de l'applicateur (12) et/ou le corps de poinçon (14), dans lequel le tube de canule (20) est partiellement inséré.

5. Applicateur dentaire selon l'une des revendications précédentes, **caractérisé en ce que** le dispositif de réception (22), en particulier l'évidement, présente une butée de profondeur (44) et que le tube de canule (20) peut être inséré dans le dispositif de réception (22) ou l'évidement jusqu'au contact avec la butée de profondeur (44).

6. Applicateur dentaire selon l'une des revendications précédentes, **caractérisé en ce qu'**un évidement du dispositif de réception (22) présente une profondeur, qui est au moins deux fois, de préférence au moins trois fois et de manière particulièrement préférée pas plus de dix fois le diamètre du tube de canule (20).

7. Applicateur dentaire selon l'une des revendications précédentes, **caractérisé en ce que** l'applicateur dentaire comprend un tube de canule (20) et le tube de canule (20) présente un diamètre avec un guide de jeu permettant un jeu d'au moins 50 µm dans l'évidement, et dans ce contexte le diamètre intérieur de l'évidement est au moins 50 µm plus grand que le diamètre extérieur du tube de canule (20).

8. Applicateur dentaire selon l'une des revendications précédentes, **caractérisé en ce que** le dispositif de réception (22) possède au moins deux butées de flexion, espacés l'une de l'autre, en particulier en ce qui concerne la profondeur de pénétration du tube de canule (20) dans le dispositif de réception (22), lesdites butées de flexion étant décalées de 180° l'une par rapport à l'autre.

9. Applicateur dentaire selon l'une des revendications précédentes, **caractérisé en ce que** le dispositif de réception (22) présente un évidement, est circulaire et s'étend en ligne droite.

10. Applicateur dentaire selon l'une des revendications précédentes, **caractérisé en ce que** le dispositif de réception (22) sur son extrémité tournée vers l'extérieur présente au moins une extrémité (42) incurvée, qui s'étend en particulier circulaire autour de l'extrémité tournée vers l'extérieur (42).

11. Applicateur dentaire selon la revendication 10, **caractérisé en ce que** l'extrémité incurvée (42) présente un rayon, qui correspond à moins au rayon de courbure minimal admissible du tube de canule (20).

12. Applicateur dentaire selon la revendication 10, **caractérisé en ce que**, vers la sortie du rayon, l'extrémité incurvée (42) peut au moins présenter un angle de rampe (43) prédéfini.
